(19) Europäisches Patentamt
European Patent Office
Office européen des brevets

(11) Veröffentlichungsnummer: **0 101 670**
**B1**

(12) **EUROPÄISCHE PATENTSCHRIFT**

(45) Veröffentlichungstag der Patentschrift: **09.03.88**

(21) Anmeldenummer: **83810366.1**

(22) Anmeldetag: **17.08.83**

(51) Int. Cl.⁴: **C 07 D 251/16**, C 07 D 239/46, C 07 D 239/42, C 07 D 251/22, C 07 D 251/46, C 07 D 251/18, C 07 D 251/52, C 07 D 239/52, C 07 D 239/48, C 07 D 239/56, C 07 D 405/12 // C07F7/10, C07F7/08

# (54) Verfahren zur Herstellung von herbiziden und pflanzenwuchsregulierenden Sulfonylharnstoffen.

(30) Priorität: **23.08.82 CH 4999/82**

(43) Veröffentlichungstag der Anmeldung: **29.02.84 Patentblatt 84/9**

(45) Bekanntmachung des Hinweises auf die Patenterteilung: **09.03.88 Patentblatt 88/10**

(84) Benannte Vertragsstaaten: **AT BE CH DE FR GB IT LI NL SE**

(56) Entgegenhaltungen:
**EP - A - 0 044 807**
**EP - A - 0 051 465**
**EP - A - 0 070 698**
**EP - A - 0 079 683**
**FR - A - 2 051 513**
**US - A - 3 435 116**

(73) Patentinhaber: **CIBA-GEIGY AG, Klybeckstrasse 141, CH-4002 Basel (CH)**

(72) Erfinder: **Szczepanski, Henry, Dr., Bodenmatt, CH-4323 Wallbach (CH)**
Erfinder: **Föry, Werner, Dr., Benkenstrasse 65, CH-4054 Basel (CH)**
Erfinder: **Meyer, Willy, Talweg 49, CH-4125 Riehen (CH)**
Erfinder: **Töpfl, Werner, Dr., Dorneckstrasse 68, CH-4143 Dornach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 101 670 B1

ACTORUM AG

**Beschreibung**

Die vorliegende Erfindung betrifft ein neues Verfahren zur Herstellung von herbiziden und pflanzenwuchsregulierenden Sulfonylharnstoffen sowie neue als Zwischenprodukte hergestellte Pyrimidinyl- und Triazinylcarbamate und Trimethylsilylamino-pyrimidine und triazine.

Die nach dem neuen erfindungsgemässen Verfahren herzustellenden Sulfonylharnstoffe sind in den US-Patenten 4 127 405, 4 301 286 und 4 302 241, den europäischen Offenlegungsschriften 23 422, 44 807, 44 808, 70 802 und 72 347 mit ihren Eigenschaften beschrieben.

Die bekannten Verfahren sind insofern nachteilig, als entweder mit Isocyanat- oder Isothiocyanat-Derivaten gearbeitet werden muss, deren Handhabung wegen der grossen Reaktivität dieser Verbindungsklasse schwierig ist, oder bei der Synthese aus Phenylcarbamaten ökologisch belastende Nebenprodukte wie z.B. Phenole anfallen.

Aus der europäischen Offenlegungsschrift 51 465 ist ein Verfahren zur Herstellung ähnlicher Sulfonylharnstoffe bekannt, das die obigen Nachteile weitgehend vermeidet. Nach diesem Verfahren werden Sulfonylharnstoffe hergestellt, indem man ein Sulfonamid in Gegenwart von mindestens äquimolaren Mengen Trimethylaluminium mit einem Pyrimidinyl- oder Triazinyl-methylcarbamat umsetzt. Dieses Verfahren ist insofern nachteilig, als das verwendete Trimethylaluminium pyrophor ist und ausserdem von Luft und Feuchtigkeit zersetzt wird.

Es ist somit Ziel der vorliegenden Erfindung, ein Verfahren bereitzustellen, dass die Verwendung schlecht handhabbarer Verbindungen und den Anfall von umweltbelastenden Nebenprodukten vermeidet.

Es wurde nun überraschenderweise festgestellt, dass die Umsetzung von Sulfonamiden mit Alkylcarbamaten auch ohne Trialkylaluminium durchgeführt werden kann.

Erfindungsgemäss wird somit vorgeschlagen, die herbiziden und pflanzenwuchsregulierenden Sulfonylharnstoffe der allgemeinen Formel I

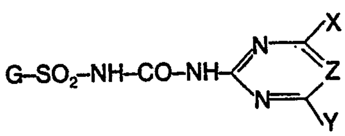
(I)

worin

G einen Rest der Formeln

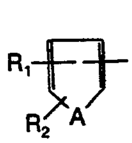
oder
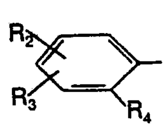
,

X $C_1$–$C_4$-Alkyl, $C_1$–$C_4$-Halogenalkyl, $C_1$–$C_4$-Alkoxy, $C_1$–$C_4$-Alkylthio, $C_1$–$C_4$-Halogenalkoxy, $C_1$–$C_4$-Alkylamino oder Di-$C_1$–$C_4$-alkylamino

Y $C_1$–$C_4$-Alkyl, $C_1$–$C_4$-Halogenalkyl, $C_1$–$C_4$-Alkoxy oder $C_1$–$C_4$-Halogenalkoxy und

Z Stickstoff oder die Methinbrücke bedeuten, worin

A für Sauerstoff, Schwefel, $-NR_5-$ oder $-C=N-$, wobei $R_5$ Wasserstoff, $C_1$–$C_4$-Alkyl oder $-CO-C_1$–$C_4$-Alkyl bedeutet,

$R_1$ für Wasserstoff, Halogen, Nitro, $-Q-C_1$–$C_4$-Alkyl, $C_1$–$C_4$-Alkyl, $CF_3$, $-SO_2$–Di–$C_1$–$C_4$-alkylamino, $-CO-Q-C_3$–$C_5$-Alkinyl oder gegebenenfalls durch Halogen, Cyan, $C_1$–$C_4$-Alkoxy oder $C_1$–$C_4$-Alkylthio substituiertes $-CO-Q-C_1$–$C_4$-Alkyl oder $-CO-Q-C_3$–$C_5$-Alkenyl,

$R_2$ für Wasserstoff, Halogen, $CF_3$, $NO_2$, $C_1$–$C_4$-Alkyl oder $C_1$–$C_4$-Alkoxy,

$R_3$ für Wasserstoff, Fluor oder Chlor,

$R_4$ für Wasserstoff, Halogen, Nitro, $CF_3$, $C_1$–$C_4$-Alkyl, $-CHR_6R_7$, $-SO_2$–Di–$C_1$–$C_4$-alkylamino, $-Q-C_3$–$C_5$-Alkinyl, $CO-T-C_3$–$C_5$-Alkinyl oder gegebenenfalls durch Halogen, Cyan, $C_1$–$C_4$-Alkoxy oder $C_1$–$C_4$-Alkylthio substituiertes $-Q-C_1$–$C_4$-Alkyl, $-Q-C_2$–$C_5$-Alkenyl, $-CO-T-C_1$–$C_4$-Alkyl oder $-CO-T-C_3$–$C_5$-Alkenyl, wobei n Null, eins oder zwei, Q ein Sauerstoff-, Schwefel-, $-SO_n-$, $-NH-$ oder $-N(C_1$–$C_4$-Alkyl)- und T eine Sauerstoff-, Schwefel-, $-NH-$ oder $-N(C_1$–$C_4$-Alkyl)-Brücke bedeuten,

$R_6$ für Wasserstoff, Chlor oder Methyl und

$R_7$ für Chlor, Cyan, Methoxy, Äthoxy oder $-SO_n-C_1$–$C_4$-Alkyl stehen, in der Weise herzustellen, dass man ein Sulfonamid der Formel II

$$G-SO_2-NH_2 \qquad (II)$$

worin G die oben angegebene Bedeutung hat, mit einem N-Pyrimidinyl- oder N-Triazinylcarbamat der Formel III

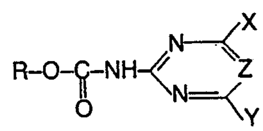
(III)

worin X, Y und Z die oben angegebene Bedeutung haben und R für Methyl oder Äthyl steht, umsetzt.

Unter Halogen ist im Rahmen der obigen Definition im allgemeinen Fluor, Chlor, Brom oder Jod zu verstehen. Bevorzugt sind Fluor und Chlor. Als Substituent des Restes G ist insbesondere Chlor und als Substituent einer Alkylgruppe, die ihrerseits Teil eines Restes sein kann, ist insbesondere Fluor bevorzugt.

Beispiele für Alkyl sind Methyl, Äthyl, n-Propyl, i-Propyl oder die isomeren Butyl. Alkyl ist dabei selbst als Substituent oder als Teil eines anderen Substituenten wie beispielsweise Alkoxy oder Alkylthio zu verstehen. Bevorzugte Alkylreste sind jeweils unverzweigte Alkylketten, insbesondere aber Methyl und Äthyl.

Bevorzugte Substituenten des Restes G sind unter den Substituenten $R_1$, $R_2$, $R_3$, $R_4$ und $R_5$ solche, die ausgewählt sind aus der Gruppe Chlor, Methoxycarbonyl, Nitro, Dimethylsulfonamyl, Trifluormethyl, Methylsulfonyl und n-Propylsulfonyl.

In der Regel sind unter Alkenyl, Allyl, 2-Butenyl, 3-Butenyl, 2-Isobutenyl, Isopropenyl, 2-Pentenyl, 3-Pentenyl und 4-Pentenyl, insbesondere aber Allyl und 4-Pentenyl zu verstehen.

Unter Alkinyl versteht man im allgemeinen Propargyl, 2-Butinyl, 3-Butinyl, Methylpropargyl, 2-Pentinyl, 3-Pentinyl und 4-Pentinyl.

Die Heterocyclen, die unter die Definition des Restes G fallen, sind Thiophen, Furan, Pyrrol und Pyridin.

Unter den Pyrimidin- und Triazinringen sind diejenigen bevorzugt, in denen X Methyl, Äthyl, Fluormethyl, Trifluormethyl, Methoxy, Äthoxy, i-Propyloxy, Methylthio, Chlor, Brom, Difluormethoxy, 2,2,2-Trifluoräthoxy, Methylamino oder Dimethylamino und Y Methyl, Methoxy oder Difluormethoxy bedeuten.

Die Carbamate der Formel III werden mit den Sulfonamiden der Formel II zur Reaktion gebracht, indem das Gemisch beider Reaktionspartner erhitzt wird, bis eine Abspaltung des Alkohols eintritt. Die Umsetzung kann dabei sowohl ohne Lösungsmittel, als auch in Gegenwart eines inerten Lösungsmittels erfolgen. Im allgemeinen wird das Reaktionsgemisch bis zum Eintritt der Abspaltungsreaktion des Alkohols aufgeheizt und bei dieser Temperatur gehalten bis ein vollständiger Umsatz erreicht ist. Die Temperatur beträgt dabei in der Regel 20° bis 200 °C, vorzugsweise 40° bis 100 °C. Als geeignete Lösungsmittel haben sich bewährt: Kohlenwasserstoffe wie Benzol, Toluol, Xylol, Mesitylen, Tetrahydronaphthalin, Decalin, Cyclohexan und höhersiedende Benzinfraktionen; Äther wie Tetrahydrofuran, Dioxan, Äthylenglykoldimethyläther, Diäthylenglykoldimethyläther und Diphenyläther; Nitrile wie Acetonitril oder Propionitril, Ketone wie Äthylmethylketon, Cyclohexanon und Aceton; Amide wie Dimethylformamid oder N-Methylpyrrolidinon und Dimethylsulfoxid.

Als Vorteil erweist sich bei der erfindungsgemässen Reaktion häufig der Zusatz einer Base. Besonders geeignete Basen sind hierfür: 1,5-Diazabicyclo[4,3,0]non-5-en (DBN) oder 1,5-Diazabicyclo[5,4,0]undec-5-en (DBU). Die Reaktionstemperaturen liegen in der Regel zwischen 20 °C und 200 °C, vorzugsweise zwischen 40 °C und 100 °C.

In einer bevorzugten Ausführungsform des erfindungsgemässen Verfahrens wird daher in der Weise vorgegangen, dass man ein Sulfonamid der Formel II in Gegenwart einer Base mit einem Carbamat der Formel III in einem inerten Lösungsmittel auf 40° bis 100 °C erhitzt bis die Abspaltungsreaktion des Alkohols abgeschlossen ist und das Produkt isoliert.

Die Ausgangsverbindungen der Formel II sind bekannt oder können analog zu bekannten Verfahren hergestellt werden.

Die N-Pyrimidinyl- und N-Triazinylcarbamate der Formel III sind z.T. bekannt. Sie werden durch Reaktion der Aminopyrimidine oder -triazine der Formel IV

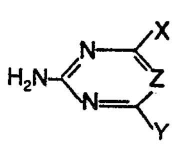 (IV)

worin X, Y und Z die oben angegebene Bedeutung haben, mit einem Kohlensäureester der Formel V

R–O–CO–OR (V)

oder einem Chlorameisensäureester der Formel VI

Cl–CO–OR (VI)

worin R Methyl oder Äthyl bedeuten, erhalten.

Neue R-Pyrimidinyl- und N-Triazinylcarbamate entsprechen den Unterformeln IIIa und IIIb

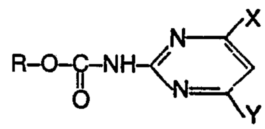 (IIIa)

worin R, X und Y die unter Formel III gegebene Bedeutung haben, mit der Massgabe, dass R Äthyl bedeutet, wenn gleichzeitig Y für Methyl und X für Methoxy stehen; und

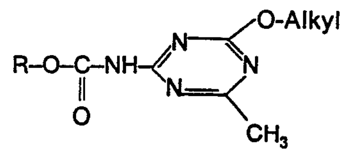 (IIIb)

worin R die unter Formel III gegebene Bedeutung hat und Alkyl für $C_1$–$C_4$-Alkyl steht.

Die neuen Verbindungen der Formeln IIIa und IIIb sind speziell für die erfindungsgemässe Synthese der Wirkstoffe der Formel I entwickelt worden, und bilden daher einen Gegenstand der vorliegenden Erfindung.

Die Umsetzung der Aminopyrimidine und -triazine der Formel IV mit den Kohlensäureestern der Formel V oder den Chlorameisensäureestern der Formel VI kann in Gegenwart eines geeigneten inerten aprotischen Lösungsmittels oder auch in Abwesenheit eines Lösungsmittels durchgeführt werden. Als vorteilhaft hat sich jedoch die Verwendung eines Lösungsmittels erwiesen. Geeignete Lösungsmittel sind Kohlenwasserstoffe wie Benzol, Toluol und Xylol; Äther wie Diäthyläther, Äthylenglykoldimethyläther, Diäthylenglykoldimethyläther, Tetrahydrofuran und Dioxan; Ketone wie Aceton, Äthylmethylketon und Cyclohexanon; und Nitrile wie Acetonitril und Propionitril. Die Umsetzung erfolgt in Gegenwart von mindestens äquimolaren Mengen einer Base. Geeignete Basen sind Carbonate wie Natrium- und Kaliumcarbonat, Hydrogencarbonate wie Natrium- und Kaliumhydrogencarbonat, Oxide wie Calcium- und Magnesiumoxid und tertiäre Amine wie Trimethylamin, Triäthylamin, Chinuclidin, Chinolin, Pyridin und Tripropylamin. Mit Vorteil wird die Base im Überschuss eingesetzt. So werden pro Mol Sulfonamid vorzugsweise 1 bis 5 Mol Base, insbesondere 1,1 bis 1,5 Mol eingesetzt. Grössere Basenüberschüsse werden besonders dann verwendet, wenn die Reaktion ohne Lösungsmittel durchgeführt wird und die Base, vorzugsweise ein flüssiges tertiäres Amin, gleichzeitig als Reaktionsmedium dient. Die Reaktionstemperaturen liegen in der Regel zwischen 0 °C und 140 °C, vorzugsweise zwischen 10 °C und 80 °C.

In einigen Fällen kann es vorteilhaft sein, die Carbamate der Formel III nicht nach dem oben skizzierten Verfahren herzustellen, sondern zur Erzielung einer höheren Ausbeute das folgende Verfahren anzuwenden.

Danach erhält man die N-Pyrimidinyl- und N-Triazinylcarbamate der Formel III, indem man ein Aminopyrimidin oder -triazin der Formel III mit einem Silylierungsmittel in die Silylaminopyrimidine oder -triazine der Formel VII

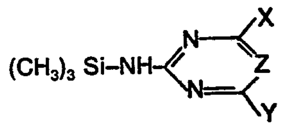

(VII)

überführt und diese mit den Chlorameisensäureestern der Formel VI umsetzt.

Die Silylierung erfolgt üblicherweise durch Erhitzen des Aminopyrimidins oder -triazins zusammen mit einem Silylierungsmittel, wie N,O-Bis-(trimethylsilyl)-acetamid, Hexamethyldisilazan oder Trimethylchlorsilan, gegebenenfalls in Gegenwart eines Katalysators wie konzentrierte Schwefelsäure oder Chlorwasserstoff in einem inerten Lösungsmittel auf 30 °C bis 150 °C, vorzugsweise auf 40 °C bis 80 °C. Geeignete Lösungsmittel sind: Kohlenwasserstoffe wie Benzol, Toluol und Xylol; Äther wie Diäthyläther, Äthylenglykoldimethyläther, Diäthyläther, Diäthylenglykoldimethyläther, Tetrahydrofuran und Dioxan; Nitrile wie Acetonitril und Propionitril; und Dimethylsulfoxid. Die weitere Reaktion der Silylaminopyrimidine oder -triazine der Formel VII mit den Chlorameisensäureestern der Formel VI zu den N-Pyrimidinyl- und N-Triazinylcarbamaten der Formel III wird durch Erwärmen auf 30 °C bis 120 °C, vorzugsweise auf 40 °C bis 80 °C bewirkt. Diese Umsetzung kann ohne Lösungsmittel oder in einem inerten Lösungsmittel wie z. B. Benzol, Toluol, Xylol, Petroläther, Cyclohexan, Diäthyläther, Dioxan, Tetrahydrofuran.

Die Zwischenprodukte der Formel VII, worin X, Y und Z die unter Formel I gegebene Bedeutung haben, mit der Massgabe, dass X für $C_1$–$C_4$-Alkoxy und Y für Methyl stehen wenn Z Stickstoff bedeutet, sind neu und speziell für das erfindungsgemässe Herstellungsverfahren entwickelt worden. Sie bilden daher ebenfalls einen Bestandteil der vorliegenden Erfindung.

Die Ausgangsmaterialien der Formeln V und VI sind bekannt und im Handel erhältlich.

Die folgenden Beispiele dienen der näheren Erläuterung der vorliegenden Erfindung.

Beispiel 1:
N-(2-Chlorphenyl-sulfonyl)-N′-(4-methoxy-6-methyl-1,3,5-triazin-2-yl)-harnstoff.

Eine Mischung von 1,0 g 2-Chlorphenylsulfonamid, 1,0 g N-(4-Methoxy-6-methyl-1,3,5-triazin-2-yl)-methylcarbamat und 1,0 g 1,5-Diazabicyclo-[4,3,0]non-5-en in 10 ml Dioxan wird für 14 Stunden bei 100 °C gerührt. Nach dem Zusatz von 50 ml Wasser wird das Gemisch mit 10%-iger Salzsäure angesäuert und mit Äthylacetat extrahiert. Durch Eindampfen der organischen Phase und Kristallisation des Rückstandes aus Methylenchlorid/Äther-Gemisch erhält man 0,75 g
N-(2-Chlorphenylsulfonyl)-N′-(4-methoxy-6-methyl-1,3,5-triazin-2-yl)-harnstoff,
Smp. 176–178 °C.

Zwischenproduktdarstellung 1:
a) 4-Methoxy-6-methyl-2-trimethylsilylamino-1,3,5-triazin.

Ein Gemisch von 238,2 g 2-Amino-4-methoxy-6-methoxy-6-methyl-1,3,5-triazin, 505 ml N,O-Bis-(trimethylsilyl)-acetamid und 1150 ml trockenem Acetonitril wird für 8 Stunden am Rückfluss gekocht. Durch fraktionierte Destillation erhält man 296,4 g 4-Methoxy-6-methyl-2-trimethylsilylamino-1,3,5-triazin, Sdp. 78 °C/0,053 mb.

b) N-(4-Methoxy-6-methyl-1,3,5-triazin-2-yl)-methylcarbamat.

Ein Gemisch von 10,6 g 4-Methoxy-6-methyl-2-trimethylsilylamino-1,3,5-triazin und 14,2 g Chlorameisensäuremethylester wird für 20 Stunden bei 65 °C gerührt. Die flüchtigen Bestandteile werden im Vakuum abgedampft und der Rückstand mit 30 ml Methylenchlorid aufgenommen. Nach Abtrennung der unlöslichen Bestandteile wird die Lösung eingedampft und der Rückstand an Kieselgel chromatographiert. Man erhält so 6,2 g N-(4-Methoxy-6-methyl-1,3,5-triazin-2-yl)-methylcarbamat, Smp. 102–104 °C.

Zwischenproduktdarstellung 2:
4-Difluormethoxy-6-methyl-2-trimethylsilyl-pyrimidin.

Eine Mischung von 21,0 g 2-Amino-4-difluormethoxy-6-methyl-pyrimidin und 0,27 ml konzentrierter Schwefelsäure wird in 90 ml trockenen Toluol auf 60 °C erwärmt und innerhalb von 10 Minuten mit einem Gemisch aus 12,5 ml Hexamethyldisilazan und 7,6 ml Trimethylchlorsilan versetzt. Die entstandene Suspension wird bei 60 °C für 24 Stunden gerührt, anschliessend auf 20 bis 25 °C abgekühlt, filtriert und fraktioniert destilliert. Man erhält so 26 g 4-Difluormethoxy-6-methyl-2-trimethylsilyl-pyrimidin, Sdp. 95°/11mb.

Zwischenproduktdarstellung 3:
a) 4-Methoxy-6-methyl-2-trimethylsilylamino-pyrimidin.

Ein Gemisch von 27,9 g 2-Amino-4-methoxy-6-methyl-pyrimidin und 48,8 g N,O-Bis-(trimethylsilyl)-acetamid wird für 7 Stunden in 250 ml Acetonitril am Rückfluss gekocht. Nach dem Abkühlen wird der unlösliche Bodensatz abgetrennt und das Filtrat fraktioniert destilliert. Man erhält so 36,3 g 4-Methoxy-6-methyl-2-trimethylsilylamino-pyrimidin, Sdp. 62–64 °C/0,026 mb.

b) N-(4-methoxy-6-methyl-pyrimidin-2-yl)-methylcarbamat.

Ein Gemisch von 10,6 g 4-Methoxy-6-methyl-2-trimethylsilylamino-pyrimidin und 47 g Chlorameisensäuremethylester wird in 120 ml Petroläther für 7 Stunden bei 50 °C gerührt. Beim Abkühlen der Lösung kristallisieren 5,5 g

N-(4-Methoxy-6-methyl-pyrimidin-2-yl)-methylcarbamat aus, Smp. 92–95 °C.

In analoger Weise werden die in den folgenden Tabellen aufgelisteten Zwischenprodukte erhalten.

Tabelle 1:

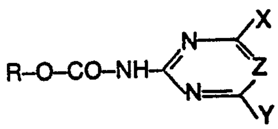

| Nr. | R | X | Y | Z | phys. Daten |
|---|---|---|---|---|---|
| 1.1 | $CH_3$ | $OCH_3$ | $CH_3$ | N | Smp. 102–104 °C |
| 1.2 | $CH_3$ | $OCH_3$ | $OCH_3$ | N | |
| 1.3 | $CH_3$ | $OCH_3$ | $CH_3$ | CH | Smp. 92– 95 °C |
| 1.4 | $CH_3$ | $OCH_3$ | $OCH_3$ | CH | |
| 1.5 | $CH_3$ | $CH_3$ | $CH_3$ | CH | Smp. 96– 98 °C |
| 1.6 | $CH_3$ | $CH_3$ | $CH_3$ | N | |
| 1.7 | $CH_3$ | $OCHF_2$ | $CH_3$ | CH | |
| 1.8 | $C_2H_5$ | $OCHF_2$ | $CH_3$ | CH | Smp. 86– 89 °C |
| 1.9 | $CH_3$ | $OCHF_2$ | $OCH_3$ | CH | |
| 1.10 | $CH_3$ | $OCHF_2$ | $OCHF_2$ | CH | |
| 1.11 | $C_2H_5$ | $OCH_3$ | $CH_3$ | N | Smp. 73– 74 °C |
| 1.12 | $C_2H_5$ | $OCH_3$ | $OCH_3$ | N | |
| 1.13 | $C_2H_5$ | $OCH_3$ | $CH_3$ | CH | |
| 1.14 | $C_2H_5$ | $OCH_3$ | $OCH_3$ | CH | |
| 1.15 | $C_2H_5$ | $CH_3$ | $CH_3$ | CH | Smp. 68– 69 °C |
| 1.16 | $CH_3$ | $C_2H_5$ | $CH_3$ | N | |

Tabelle 2:

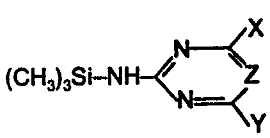

| Nr. | X | Y | Z | phys. Daten |
|---|---|---|---|---|
| 2.1 | $OCH_3$ | $CH_3$ | N | Sdp. 78 °C/0.053 mb |
| 2.2 | $OCHF_2$ | $CH_3$ | CH | Sdp. 95 °C/11 mb |
| 2.3 | $OCH_3$ | $CH_3$ | CH | Sdp. 62–64 °C/0.026 mb |
| 2.4 | $OCH_3$ | $OCH_3$ | N | |
| 2.5 | $OCH_3$ | $CH_2F$ | CH | |
| 2.6 | $OCHF_2$ | $CH_3$ | N | |
| 2.7 | $OCH_3$ | $OCH_3$ | CH | |
| 2.8 | $CH_3$ | $CH_3$ | N | |
| 2.9 | $CH_3$ | $CH_3$ | CH | |
| 2.10 | $OCHF_2$ | $OCH_3$ | CH | |
| 2.11 | $OCHF_2$ | $OCHF_2$ | CH | |

**Patentansprüche**

1. Verfahren zur Herstellung von N-Arylsulfonyl-N'-pyrimidinyl und -triazinylharnstoffen der allgemeinen Formel I

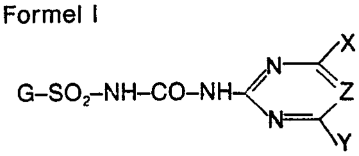 (I)

worin

G einen Rest der Formeln

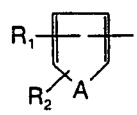 oder 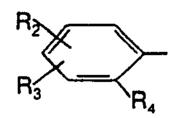 ,

X $C_1$–$C_4$-Alkyl, $C_1$–$C_4$-Halogenalkyl, $C_1$–$C_4$-Alkoxy, $C_1$–$C_4$-Alkylthio, $C_1$–$C_4$-Halogenalkoxy, $C_1$–$C_4$-Alkylamino oder Di-$C_1$–$C_4$-alkylamino,

Y $C_1$–$C_4$-Alkyl, $C_1$–$C_4$-Halogenalkyl, $C_1$–$C_4$-Alkoxy oder $C_1$–$C_4$-Halogenalkoxy und

Z Stickstoff oder die Methinbrücke bedeuten, worin

A für Sauerstoff, Schwefel, $-NR_5-$ oder $-C=N-$, wobei $R_5$ Wasserstoff, $C_1$–$C_4$-Alkyl oder $-CO-C_1-C_4$-Alkyl bedeutet,

$R_1$ für Wasserstoff, Halogen, Nitro, $-Q-C_1-C_4$-Alkyl, $C_1$–$C_4$-Alkyl, $CF_3$, $-SO_2$–Di–$C_1$–$C_4$-alkylamino, $-CO-Q-C_3-C_5$-Alkinyl oder gegebenenfalls durch Halogen, Cyan, $C_1$–$C_4$-Alkoxy oder $C_1$–$C_4$-Alkylthio substituiertes $-CO-Q-C_1-C_4$-Alkyl oder $-CO-Q-C_3-C_5$-Alkenyl,

$R_2$ für Wasserstoff, Halogen, $CF_3$, $NO_2$, $C_1$–$C_4$-Alkyl oder $C_1$–$C_4$-Alkoxy,

$R_3$ für Wasserstoff, Fluor oder Chlor,

$R_4$ für Wasserstoff, Halogen, Nitro, $CF_3$, $C_1$–$C_4$-Alkyl, $-CHR_6R_7$, $-SO$–Di–$C_1$–$C_4$-alkylamino, $-Q-C_3-C_5$-Alkinyl, $-CO-T-C_3-C_5$-Alkinyl oder gegebenenfalls durch Halogen, Cyan, $C_1$–$C_4$-Alkoxy oder $C_1$–$C_4$-Alkylthio substituiertes $-Q-C_1-C_4$-Alkyl, $-Q-C_2-C_5$-Alkenyl, $-CO-T-C_1-C_4$-Alkyl oder $-CO-T-C_3-C_5$-Alkenyl, wobei n Null, eins oder zwei, Q ein Sauerstoff-, Schwefel-, $-SO_n-$, $-NH-$ oder $-N(C_1-C_4$-Alkyl) und T eine Sauerstoff-, Schwefel-, $-NH-$ oder $-N(C_1-C_4$-Alkyl)-Brücke bedeuten,

$R_6$ für Wasserstoff, Chlor oder Methyl und

$R_7$ für Chlor, Cyan, Methoxy, Äthoxy oder $-SO_n-C_1-C_4$-Alkyl stehen, dadurch gekennzeichnet, dass man ein Sulfonamid der Formel II

$$G-SO_2-NH_2 \qquad (II)$$

worin G die oben angegebene Bedeutung hat, mit einem N-Pyrimidinylcarbamat oder N-Triazinylcarbamat der Formel III

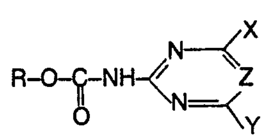 (III)

worin X, Y und Z die oben angegebene Bedeutung haben und R für Methyl oder Äthyl steht, umsetzt.

2. Verfahren gemäss Anspruch 1, dadurch gekennzeichnet, dass die Umsetzung bei einer Temperatur von 20 °C bis 200 °C erfolgt.

3. Verfahren gemäss Anspruch 2, dadurch gekennzeichnet, dass die Reaktionstemperatur 40 °C bis 100 °C beträgt.

4. Verfahren gemäss Anspruch 1, dadurch gekennzeichnet, dass die Umsetzung in einem inerten Lösungsmittel durchgeführt wird.

5. Verfahren gemäss Anspruch 4, dadurch gekennzeichnet, dass das Lösungsmittel ausgewählt ist aus der Gruppe Kohlenwasserstoffe, Äther, Nitrile, Ketone oder Dimethylsulfoxid.

6. Verfahren gemäss Anspruch 1, dadurch gekennzeichnet, dass die Umsetzung in Gegenwart einer Base erfolgt.

7. Verfahren gemäss Anspruch 6, dadurch gekennzeichnet, dass die Base ausgewählt ist aus der Gruppe 1,5-Diazabicyclo[4,3,0]non-5-en oder 1,5-Diazabicyclo[5,4,0]undec-5-en.

8. Verfahren gemäss Anspruch 1, dadurch gekennzeichnet, dass man das Sulfonamid der Formel II mit einem Carbamat der Formel III in Gegenwart einer Base in einem inerten Lösungsmittel auf 40 bis 100 °C erhitzt bis die Abspaltungsreaktion des Alkohols abgeschlossen ist und das Produkt isoliert.

9. Trimethylsilylamino-pyrimidine und triazine der allgemeinen Formel VII

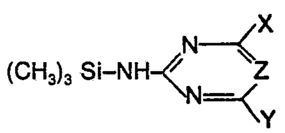

(VII)

worin X, Y und Z die unter Formel I im Anspruch 1 gegebene Bedeutung haben, mit der Massgabe, dass X für $C_1$–$C_4$-Alkoxy und Y für Methyl stehen wenn Z Stickstoff bedeutet.

10. N-Pyrimidinylcarbamat der allgemeinen Formel IIIa

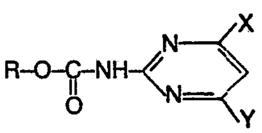

(IIIa)

worin R, X und Y die unter Formel III im Anspruch 1 gegebene Bedeutung haben, mit der Massgabe, dass R Äthyl bedeutet, wenn gleichzeitig Y für Methyl und X für Methoxy stehen.

11. N-Triazinylcarbamate der allgemeinen Formel IIIb

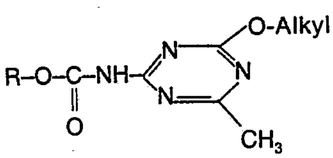

(IIIb)

worin R die unter Formel III im Anspruch 1 gegebene Bedeutung hat und Alkyl für $C_1$–$C_4$-Alkyl steht.

## Claims

1. A process for producing N-arylsulfonyl-N′-pyrimidinyl- and -triazinylureas of the general formula I

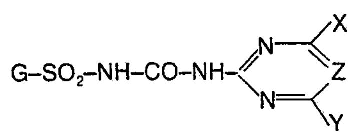

(I)

wherein

G is a radical of the formula

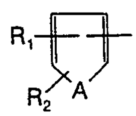 or 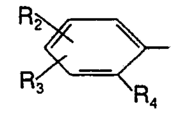

X is $C_1$–$C_4$-alkyl, $C_1$–$C_4$-haloalkyl, $C_1$–$C_4$-alkoxy, $C_1$–$C_4$-alkylthio, $C_1$–$C_4$-haloalkoxy, $C_1$–$C_4$-alkylamino, or di-$C_1$–$C_4$-alkylamino,

Y is $C_1$–$C_4$-alkyl, $C_1$–$C_4$-haloalkyl, $C_1$–$C_4$-alkoxy or $C_1$–$C_4$-haloalkoxy, and

Z is nitrogen or the methine bridge, wherein

A is oxygen, sulfur, $-NR_5-$, or $-C{=}N-$, $R_5$ being hydrogen, $C_1$–$C_4$-alkyl or $-CO-C_1-C_4$-alkyl,

$R_1$ is hydrogen, halogen, nitro, $-Q-C_1-C_4$-alkyl, $C_1$–$C_4$-alkyl, $CF_3$, $-SO_2-$di-$C_1$–$C_4$-alkylamino or $-CO-Q-C_3-C_5$-alkynyl; or $-CO-Q-C_1-C_4$-alkyl or $-CO-Q-C_3-C_5$-alkenyl each unsubstituted or substituted by halogen, cyano, $C_1$–$C_4$-alkoxy or $C_1$–$C_4$-alkylthio,

$R_2$ is hydrogen, halogen, $CF_3$, $NO_2$, $C_1$–$C_4$-alkyl or $C_1$–$C_4$-alkoxy,

$R_3$ is hydrogen, fluorine or chlorine,

$R_4$ is hydrogen, halogen, nitro, $CF_3$, $C_1$–$C_4$-alkyl, $-CHR_6R_7$, $-SO_2-$di-$C_1$–$C_4$-alkylamino, $-Q-C_3-C_5$-alkynyl or $-CO-T-C_3-C_5$-alkynyl; or $-Q-C_1-C_4$-alkyl, $-Q-C_2-C_5$-alkenyl, $-CO-T-C_1-C_4$-alkyl or $-CO-T-C_3-C_5$-alkenyl each of which is unsubstituted or substituted by halogen, cyano, $C_1$–$C_4$-alkoxy or $C_1$–$C_4$-alkylthio, n being zero, one or two, Q being an oxygen, sulfur, $-SO_n-$, $-NH-$ or $-N(C_1-C_4\text{-alkyl})-$, and T being an oxygen, sulfur, $-NH-$ or $-N(C_1-C_4\text{-alkyl})$-bridge,

$R_6$ is hydrogen, chlorine or methyl, and

$R_7$ is chlorine, cyano, methoxy, ethoxy or $-SO_n-C_1-C_4$-alkyl, characterised in that a sulfonamide of the formula II

$$G{-}SO_2{-}NH_2 \qquad (II),$$

wherein G has the meaning defined above, is reacted with an N-pyrimidinylcarbamate or N-triazinylcarbamate of the formula III

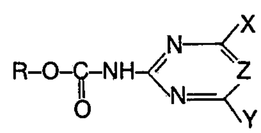

(III)

wherein X, Y and Z have the meanings defined above, and R is methyl or ethyl.

2. A process according to Claim 1, characterised in that the reaction is performed at a temperature of 20° to 200 °C.

3. A process according to Claim 2, characterised in that the reaction temperature is 40° to 100 °C.

4. A process according to Claim 1, characterised in that the reaction is performed in an inert solvent.

5. A process according to Claim 4, characterised in that the solvent is selected from the group comprising hydrocarbons, ethers, nitriles, ketones or dimethyl sulfoxide.

6. A process according to Claim 1, characterised in that the reaction is performed in the presence of a base.

7. A process according to Claim 6, characterised in that the base is selected from the group comprising 1,5-diazabicyclo[4,3,0]non-5-ene and 1,5-diazabicyclo[5,4,0]undec-5-ene.

8. A process according to Claim 1, characterised in that the sulfonamide of the formula II is heated with a carbamate of the formula III in the presence of a base in an inert solvent at 40° to 100 °C until the reaction by which the alcohol is detached is completed, and the product is then isolated.

9. Trimethylsilylamino-pyrimidines and -triazines of the general formula VII

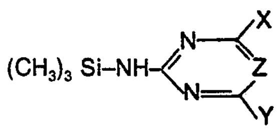 (VII)

wherein X, Y and Z have the meanings defined under the formula I in Claim 1, with the proviso that X is $C_1$–$C_4$-alkoxy and Y is methyl when Z is nitrogen.

10. N-Pyrimidinylcarbamates of the general formula IIIa

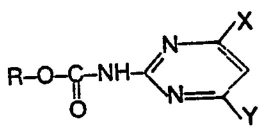 (IIIa)

wherein R, X and Y have the meanings defined under the formula III in Claim 1, with the proviso that R is ethyl when at the same time Y is methyl and X is methoxy.

11. N-Triazinylcarbamates of the general formula IIIb

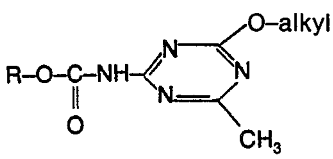 (IIIb)

wherein R has the meaning defined under the formula III in claim 1, and alkyl denotes $C_1$–$C_4$-alkyl.

**Revendications**

1. Procédé de préparation de N-arylsulfonyl-N'-pyrimidinyl- et -triazinylurées de formule générale I

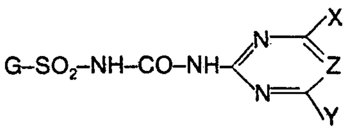 (I)

dans laquelle

G représente un radical de formules

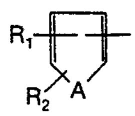 ou 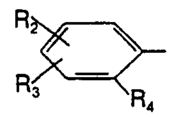

X représente un alcoyle en $C_1$ à $C_4$, halogénalcoyle en $C_1$ à $C_4$, alcoxy en $C_1$ à $C_4$, alcoylthio en $C_1$ à $C_4$, halogénalcoxy en $C_1$ à $C_4$, alcoylamino en $C_1$ à $C_4$ ou di-alcoylamino en $C_1$ à $C_4$,

Y représente un alcoyle en $C_1$ à $C_4$, halogénalcoyle en $C_1$ à $C_4$, alcoxy en $C_1$ à $C_4$ ou halogénalcoxy en $C_1$ à $C_4$ et

Z représente un azote ou le pont méthine, où

A représente un oxygène, un soufre, $-NR_5-$ ou $-C=N-$, où $R_5$ représente un hydrogène, un alcoyle en $C_1$ à $C_4$ ou CO-alcoyle en $C_1$–$C_4$,

$R_1$ représente un hydrogéne, halogène, nitro, –Q–alcoyle en $C_1$ à $C_4$, alcoyle en $C_1$ à $C_4$, $CF_3$, $-SO_2$–di–alcoylamino en $C_1$ à $C_4$, –CO–Q–alcynyle en $C_3$ à $C_5$ ou un –CO–Q–alcoyle en $C_1$ à $C_4$ ou –CO–Q–alcényle en $C_3$ à $C_5$ éventuellement substitué par un halogène, cyano, alcoxy en $C_1$ à $C_4$ ou alcoylthio en $C_1$ à $C_4$,

$R_2$ représente un hydrogène, halogène, $CF_3$, $NO_2$, alcoyle en $C_1$ à $C_4$ ou alcoxy en $C_1$ à $C_4$,

$R_3$ représente un hydrogène, un fluor ou un chlore,

$R_4$ représente un hydrogène, halogène, nitro, $CF_3$, alcoyle en $C_1$ à $C_4$, $-CHR_6-R_7$, $-SO_2$–di–alcoylamino en $C_1$ à $C_4$, –Q–alcynyle en $C_3$ à $C_5$, –CO–T–alcynyle en $C_3$ à $C_5$ ou un –Q–alcoyle en $C_1$ à $C_4$, –Q–alcényle en $C_2$ à $C_5$, –CO–T–alcoyle en $C_1$ à $C_4$ ou –CO–T–alcényle en $C_3$ à $C_5$ éventuellement substitué par un halogène, un cyano, un alcoxy en $C_1$ à $C_4$ ou un alcoylthio en $C_1$ à $CH_4$, où n vaut 0, 1 ou 2, Q représente un pont oxygène, soufre, $-SO_n-$, NH–, ou N(alcoyle en $C_1$ à $C_4$)– et T représente un pont oxygène, soufre, –NH– ou –N(alcoyle en $C_1$ à $C_4$),

$R_6$ représente un hydrogène, un chlore ou un éthyle et

$R_7$ représente un chlore, cyano, méthoxy, éthoxy ou $-SO_n$-alcoyle en $C_1$ à $CH_4$, caractérisé en ce qu'on fait réagir un sulfonamide de formule II

$$G-SO_2-NH_2 \quad (II)$$

où G a la signification donnée ci-dessus, avec un N-pyrimidinylcarbamate ou N-triazinylcarbamate de formule III

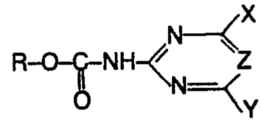 (III)

où X, Y et Z ont la signification donnée ci-dessus et R représente un méthyle ou un éthyle.

2. Procédé selon la revendication 1, caractérisé en ce qu'on conduit la réaction à une température de 20 °C à 200 °C.

3. Procédé selon la revendication 2, caractérisé en ce que la température de la réaction s'élève à 40 °C à 100 °C.

4. Procédé selon la revendication 1, caractérisé

en ce qu'on conduit la réaction dans un solvant inerte.

5. Procédé selon la revendication 4, caractérisé en ce que le solvant est choisi dans le groupe hydrocarbures, éthers, nitriles, cétones ou diméthylsulfoxide.

6. Procédé selon la revendication 1, caractérisé en ce que la réaction est conduite en présence d'une base.

7. Procédé selon la revendication 6, caractérisé en ce que la base est choisie dans le groupe 1,5-diazabicyclo[4,3,0]non-5-ène ou 1,5-diazabicyclo[5,4,0]undéc-5-ène.

8. Procédé selon la revendication 1, caractérisé en ce qu'on chauffe le sulfonamide de formule II avec un carbamate de formule III en présence d'une base dans un solvant inerte entre 40 °C et 100 °C jusqu'à ce que la réaction de séparation de l'alcool soit terminée et en ce qu'on isole le produit.

9. Triméthylsilylamino-pyrimidines et triazines de formule générale VII

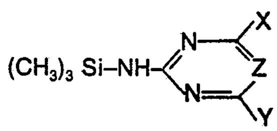

(VII)

où X, Y et Z ont la signification donnée sous la formule I dans la revendication 1, avec cette précision que X représente un alcoxy en $C_1$ à $C_4$ et Y un méthyle lorsque Z représente un azote.

10. N-pyrimidinylcarbamates de formule générale IIIa

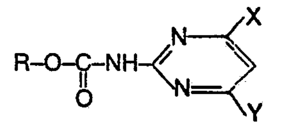

(IIIa)

où R, X et Y ont la signification donnée sous la formule III dans la revendication 1, avec cette précision que R représente un éthyle lorsque simultanément Y représente un méthyle et X un méthoxy.

11. N-triazinylcarbamates de formule générale IIIb

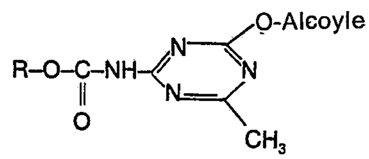

(IIIb)

où R a la signification donnée sous la formule III dans la revendication 1 et alcoyle représente un alcoyle en $C_1$ à $C_4$.